# EUROPEAN PATENT SPECIFICATION

(11) **EP 3 766 522 B1**
(45) Date of publication and mention of the grant of the patent: **23.03.2022**
(21) Application number: 20196030.9
(22) Date of filing: 21.12.2012
(51) Int. Cl.: A61K 51/04, C07B 59/00, A61K 47/12, A61K 9/08, A61K 101/02

(54) **AUTOMATED METHOD FOR THE PREPARATION OF 18F-FLUCICLOVINE COMPOSITIONS**
AUTOMATISIERTE VERFAHREN ZUR HERSTELLUNG VON 18F-FLUCICLOVINE-ZUSAMMENSETZUNGEN
MÉTHODE AUTOMATISÉE DE PRÉPARATION DE COMPOSITIONS DE LA 18F-FLUCICLOVINE

(30) Priority: 21.12.2011 WO PCT/EP2011/073670
(43) Date of publication of application: 20.01.2021
(62) Divisional of application: 12806500.0
(73) Proprietor: GE Healthcare Limited, Chalfont St. Giles, Buckinghamshire HP8 4SP (GB)
(72) Inventor: ROMOREN, Kristine, 0485 Oslo (NO); RYAN, Olav, 0411 Oslo (NO)
(74) Representative: Coles, Andrea Birgit

(56) References cited:
- EP-A1- 2 119 458
- WO-A1-03/090789
- WO-A1-2012/089594
- WO-A1-2014/023775
- ANDERS SVADBERG ET AL: "Degradation of acetonitrile in eluent solutions for [18F]fluoride PET chemistry: impact on radiosynthesis of [18F]FACBC and [18F]FDG", JOURNAL OF LABELLED COMPOUNDS AND RADIOPHARMACEUTICALS, vol. 55, no. 3, 5 March 2012 (2012-03-05), pages 97-102, XP55079899, ISSN: 0362-4803, DOI: 10.1002/jlcr.1956

## Description

### Technical Field of the Invention

The present invention relates to a drug product composition and in particular to a composition comprising a positron emission tomography (PET) tracer. The composition of the present invention has certain advantages over prior art formulations.

### Description of Related Art

The non-natural amino acid [¹⁸F]-1-amino-3-fluorocyclobutane-1-carboxylic acid ([¹⁸F]FACBC, also known as [¹⁸F]-Fluciclovine) is taken up specifically by amino acid transporters and has shown promise for tumour imaging with positron emission tomography (PET).

In radioactive diagnostic imaging agents, a problem often arises such that compounds decompose by self-radiation during delivery of the agents so as to cause decrease in radiochemical purity due to so-called radiolysis. In PET tracers comprising nuclides such as ¹¹C and ¹⁸F, radiolysis often becomes more problematic since the half-life of the nuclides used therein is relatively short, e.g. as compared with nuclides used in single photon emission tomography (SPECT) such as ^{99m}Tc, and thus radioactivity upon shipment must be set larger than SPECT agents, thereby making the resulting radiation energy thereof higher.

Various methods for inhibiting radiolysis in PET tracers have been examined. For example in compositions comprising [¹⁸F]-fluorodeoxyglucose ([¹⁸F]FDG). WO 2003/090789 discloses a method of reducing the radiolysis of [¹⁸F]FDG by adding a weak acid-based buffer to an [¹⁸F]FDG solution. WO 2004/043497 discloses adding ethanol to a [¹⁸F]FDG solution to obtain a composition of [¹⁸F]-FDG having improved stability.

In the case of [¹⁸F]FACBC different strategies have been adopted. EP 2106808 (A1) discloses that for a composition comprising [¹⁸F]FACBC, when the pH value is not more than 5.9, stability thereof is maintained even if there exist no pharmaceutical additives or buffers that prevent radiolysis.

EP 2080526 (A1) discloses that radiolysis can be inhibited by adding a sugar lactone such as ascorbic acid and glucono-o-lactone to [¹⁸F]FACBC. An exemplary composition taught by EP 2080526 (A1) has a radioactivity of 1.4GBq in about 2 mL and contains the sugar lactone in a proportion of 10mmol/mL immediately after production providing a radioactivity of 50 to 225 MBq when the agent is used, sufficient for PET imaging in adults. It was also disclosed that ascorbic acid at concentrations of 0.5-10.0 µmol/mL can inhibit decomposition of [¹⁸F]FACBC solution. In this case, radiolysis was inhibited at a concentration of 700 MBq/mL at maximum.

EP 2119458 (A1) discloses a method to prepare a stabilised formulation of [¹⁸F]FACBC comprising diluting a solution of [¹⁸F]FACBC and then adding an acid in an amount sufficient to adjust the pH of the solution to 2.0-5.9. Suitable acids disclosed are ascorbic acid, benzoic acid, hydrochloric acid, acetic acid, citric acid, gentisic acid, and oxalic acid, with hydrochloric acid preferred. EP 2119458 (A1) also discloses that a sugar alcohol such as erythritol xylitol, sorbitol or mannitol can be added as a further additive to inhibit radiolysis and improve stability.

In these known [¹⁸F]FACBC compositions radiostability is maintained by adjusting pH within a relatively wide range using an acid and/or including a suitable additive. Adjustment of pH using an acid rather than using a buffer has the advantage that the ionic strength of the composition is lower.

### Summary of the Invention

The present invention provides a pharmaceutical composition comprising [¹⁸F]FACBC having certain advantages over known compositions comprising [¹⁸F]FACBC. Also provided by the present invention is a method to obtain the composition of the invention. The composition of the present invention is resistant to degradation, can be autoclaved or diluted in saline (i.e. 0.9 % NaCl), and still maintain its pH in a narrow range. Furthermore, the pharmaceutical composition of the present invention does not require any radiostabiliser in order to maintain good radiostability over its shelf-life.

### Detailed Description of the Invention

The present invention in one aspect provides a pharmaceutical composition of ¹⁸F-FACBC characterised in that said composition:
(i) comprises 50-100 mM citrate buffer; and,
(ii) has a pH of 4.0-5.0.

The term "pharmaceutical composition" refers to a composition comprising a pharmaceutical together with a biocompatible carrier in a form suitable for mammalian administration. A "biocompatible carrier" is a fluid, especially a liquid, in which a pharmaceutical is suspended or dissolved, such that the composition is physiologically tolerable, i.e. can be administered to the mammalian body without toxicity or undue discomfort. The biocompatible carrier is suitably an injectable carrier liquid such as sterile, pyrogen-free water for injection or an aqueous solution such as saline.

The pharmaceutical composition of the invention preferably 60-90 mM citrate buffer, most preferably 75-85 mM citrate buffer.

The pharmaceutical composition of the invention preferably has a pH of 4.1-4.5, most preferably 4.3-4.4.

The pharmaceutical composition of the invention preferably has an end of synthesis (EOS) radioactive concentration (RAC) of at least 1000 MBq/mL, alternatively at least 1500 MBq/ml.

The term "end of synthesis" refers to the point in time when the labelled compound is collected in the product collection vial.

The pharmaceutical composition of the present invention has a favourable impurity profile, with the main non-radioactive impurities being 1-amino-3-hydroxyl-cyclobutane-1-carboxylic acid (hydroxyl-ACBC), 1-amino-3-fluoro-cyclobutane-1-carboxylic acid (FACBC) and 1-amino-3-chloro-cyclobutane-1-carboxylic acid (chloro-ACBC).

It is preferred that there is not more than 150 µg/mL hydroxyl-ACBC, most preferably not more than 80 µg/mL hydroxyl-ACBC.

It is preferred that there is not more than 0.15 µg/mL FACBC, most preferably not more than 0.10 µg/mL FACBC.

It is preferred that there is not more than 2.0 µg/mL chloro-ACBC, most preferably not more than 1.0 µg/mL chloro-ACBC.

The term "not more than" should be understood to mean any amount less than the quoted quantity. Therefore not more than 100 µg/mL means any amount between 0-100 µg/mL, and in an *ideal* embodiment of the composition of the present invention there would be zero µg/mL of each impurity present in the composition of the invention. However, in reality, zero µg/mL of an impurity might not be achievable and it is more likely that at least a trace amount of the impurity remains in the composition, i.e. in the case of hydroxyl-ACBC the term not more than 150 µg/mL covers e.g. 50-150 µg/mL, not more than 0.10 µg/mL for FACBC covers e.g. 0.05-0.10 µg/mL, and not more than 1.0 µg/mL chloro-ACBC covers e.g. 0.25-1.0 µg/mL.

An advantage of the composition of the present invention is that the pH, stability and impurity profile can be kept within a very narrow range over a long shelf-life, at high activities, and when manipulated e.g. by autoclaving or by dilution with 0.9% saline.

In a preferred embodiment, the pharmaceutical composition of the invention does not comprise a radiostabiliser. It is common for pharmaceutical compositions comprising radioactive pharmaceuticals to include a radiostabiliser. For example, known pharmaceutical compositions of [¹⁸F]FACBC include a sugar alcohol or a sugar lactone. EP 2080526 (A1) discloses that radiolysis can be inhibited by adding a sugar lactone such as ascorbic acid and glucono-o-lactone to [¹⁸F]FACBC, and EP 2119458 (A1) discloses that a sugar alcohol such as erythritol xylitol, sorbitol or mannitol can be added as an additive to inhibit radiolysis and improve stability. No such radiostabiliser is required in the radiopharmaceutical composition of the present invention in order to maintain a shelf-life of up to around 10 hours.

In another aspect the present invention provides a method to obtain a radiopharmaceutial composition wherein said composition is as defined hereinabove, and wherein said method comprises:
(i) reacting with a suitable source of [¹⁸F]fluoride a precursor compound of Formula I: 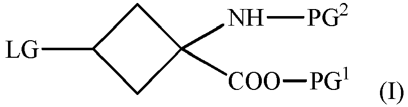 wherein:
   LG is a leaving group;
   PG¹ is a carboxy protecting group; and,
   PG² is an amine protecting group;
   to obtain a compound of Formula II: 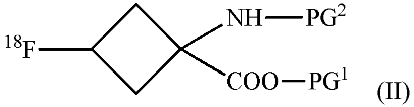 wherein PG¹ and PG² are as defined for Formula II;
(ii) reacting said compound of Formula II with a PG¹ deprotecting agent to obtain a compound of Formula III: 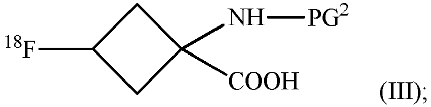 wherein PG² is as defined for Formula I;
(iii) reacting said compound of Formula III with a PG² deprotecting agent to obtain [¹⁸F]FACBC;
(iv) formulating said [¹⁸F]FACBC with citrate buffer to obtain said pharmaceutical composition.

The "source of [¹⁸F]fluoride" suitable for use in the invention is normally obtained as an aqueous solution from the nuclear reaction ¹⁸O(p,n)¹⁸F. In order to increase the reactivity of fluoride and to reduce or minimise hydroxylated by-products resulting from the presence of water, water is typically removed from [¹⁸F]-fluoride prior to the reaction, and fluorination reactions are carried out using anhydrous reaction solvents (Aigbirhio et al 1995 J Fluor Chem; 70: 279-87). A further step that is used to improve the reactivity of [¹⁸F]-fluoride for radiofluorination reactions is to add a cationic counterion prior to the removal of water. Suitably, the counterion should possess sufficient solubility within the anhydrous reaction solvent to maintain the solubility of the [¹⁸F]-fluoride. Therefore, counterions that are typically used include large but soft metal ions such as rubidium or caesium, potassium complexed with a cryptand such as Kryptofix^{™}, or tetraalkylammonium salts, wherein potassium complexed with a cryptand such as Kryptofix^{™}, or tetraalkylammonium salts are preferred.

A "precursor compound" comprises a non-radioactive derivative of a radiolabelled compound, designed so that chemical reaction with a convenient chemical form of the detectable label occurs site-specifically; can be conducted in the minimum number of steps (ideally a single step); and without the need for significant purification (ideally no further purification), to give the desired radiolabelled compound. Such precursor compounds are synthetic and can conveniently be obtained in good chemical purity.

A suitable "leaving group" in the context of the present invention is a chemical group that can be displaced by nucleophilic displacement reaction with fluoride ion. These are well-known in the art of synthetic chemistry. In some embodiments the leaving group of the present invention is a linear or branched C₁₋₁₀ haloalkyl sulfonic acid substituent, a linear or branched C₁₋₁₀ alkyl sulfonic acid substituent, a fluorosulfonic acid substituent, or an aromatic sulfonic acid substituent. In other embodiments of the invention the leaving group is selected from methanesulfonic acid, toluenesulfonic acid, nitrobenzenesulfonic acid, benzenesulfonic acid, trifluoromethanesulfonic acid, fluorosulfonic acid, and perfluoroalkylsulfonic acid. In some embodiments the leaving group is either methanesulfonic acid, trifluoromethanesulfonic acid or toluenesulfonic acid and in another embodiment the leaving group is trifluoromethanesulfonic acid.

The term "protecting group" refers to a group which inhibits or suppresses undesirable chemical reactions, but which is designed to be sufficiently reactive that it may be cleaved from the functional group in question to obtain the desired product under mild enough conditions that do not modify the rest of the molecule. Protecting groups are well known to those skilled in the art and are described in 'Protective Groups in Organic Synthesis', Theorodora W. Greene and Peter G. M. Wuts, (Fourth Edition, John Wiley & Sons, 2007).

The PG¹ "carboxy protecting group" is preferably linear or branched C₁₋₁₀ alkyl chain or an aryl substituent. The term "alkyl" used either alone or as part of another group is defined as any straight, branched or cyclic, saturated or unsaturated CₙH₂ₙ₊₁ group. The term "aryl" refers to any C₆₋₁₄ molecular fragment or group which is derived from a monocyclic or polycyclic aromatic hydrocarbon, or a monocyclic or polycyclic heteroaromatic hydrocarbon. In one embodiment of the method of the invention PG¹ is selected from methyl, ethyl, t-butyl and phenyl. In another embodiment of the invention PG¹ is methyl or ethyl and in yet another embodiment PG¹ is ethyl.

The PG² "amine protecting group" suitably prevents reaction between ¹⁸F and the amino group in the process of providing the compound of Formula II. Examples of suitable amine protecting groups include various carbamate substituents, various amide substituents, various imide substituents, and various amine substituents. Preferably, the amine protecting group is selected from the group consisting of linear or branched C₂₋₇ alkyloxycarbonyl substituents, linear or branched C₃₋₇ alkenyloxycarbonyl substituents, C₇₋₁₂ benzyloxycarbonyl substituents that may have a modifying group, C₂₋₇ alkyldithiooxycarbonyl substituents, linear or branched C₁₋₆ alkylamide substituents, linear or branched C₂₋₆ alkenylamide substituents, C₆₋₁₁ benzamide substituents that may have a modifying group, C₄₋₁₀ cyclic imide substituents, C₆₋₁₁ aromatic imine substituents that may have a substituent, linear or branched C₁₋₆ alkylamine substituents, linear or branched C₂₋₆ alkenylamine substituents, and C₆₋₁₁ benzylamine substituents that may have a modifying group. In some embodiments of the invention PG² is selected from t-butoxycarbonyl, allyloxycarbonyl, phthalimide, and N-benzylideneamine. In other embodiments PG² is selected from t-butoxycarbonyl or phthalimide. In one embodiment of the invention PG² is t-butoxycarbonyl.

The term "reacting" refers to bringing two or more chemical substances (typically referred to in the art as "reactants" or "reagents") together to result in a chemical change in one or both/all of the chemical substances.

A "PG¹ deprotecting agent" is a reagent capable of removing the carboxy protecting group PG¹ from the compound of Formula II during the reacting step (b). Suitable such carboxy deprotecting agents are well-known to the skilled person (see Greene and Wuts, *supra*) and may be either an acid or an alkaline solution. The concentration of the PG¹ deprotecting agent is not limited as long as it is sufficient to remove the carboxy protecting group PG¹ and does not have an effect on the final purity or results in an incompatibility with any container used. Preferably the PG¹ deprotecting agent is an alkaline solution. In certain embodiments the PG¹ deprotecting agent is a sodium hydroxide or a potassium hydroxide solution and in a preferred embodiment is a sodium hydroxide solution, for example of 0.5-2.0M. The reacting step is enabled by closing the outlet of the SPE column so that the PG¹ deprotecting agent is retained therein for a specified amount of time. The temperature and the duration of this reacting step need to be sufficient to permit removal of the PG¹ carboxy deprotecting group. In certain embodiments the reacting step is carried out at room temperature and for a duration of between 1-5 minutes.

The "PG² deprotecting agent" is a reagent capable of removing the amine protecting group PG² from the compound of Formula III during the reacting step (e). Suitable such amine deprotecting agents are well-known to the skilled person (see Greene and Wuts, *supra*) and may be either an acid or an alkaline solution. The concentration of the PG² deprotecting agent is not limited as long as it is sufficient to remove the carboxy protecting group PG². Preferably the PG² deprotecting agent is an acid solution. A suitable acid preferably includes an acid selected from inorganic acids such as hydrochloric acid, sulfuric acid and nitric acid, and organic acids such as perfluoroalkyl carboxylic acid, e.g. trifluoroacetic acid. In certain embodiments, the PG² deprotecting agent is hydrochloric acid, and in other embodiments when HCl is used as PG² deprotecting agent it is at a concentration of 1.0-4.0M. Reacting step (e) is preferably carried out with heat to allow the removal of PG² reaction to proceed more rapidly. The reaction time depends on the reaction temperature or other conditions. For example, when the reacting step (e) is performed at 60°C, a sufficient reaction time is 5 minutes.

Precursor compounds of Formula I may be obtained by following or adapting methods known in the art, such as for example described by McConathy et al (2003 Appl Radiat Isotop; 58: 657-666) or by Shoup and Goodman (1999 J Label Comp Radiopharm; 42: 215-225).

In a preferred aspect, the [¹⁸F]-FACBC is trans-1-amino-3-[¹⁸F]-fluorocyclobutanecarboxylic acid (*anti*-[¹⁸F]-FACBC): 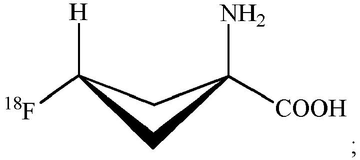 said compound of Formula I is a compound of Formula Ia: 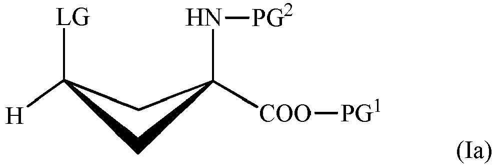 said compound of Formula II is a compound of Formula IIa: 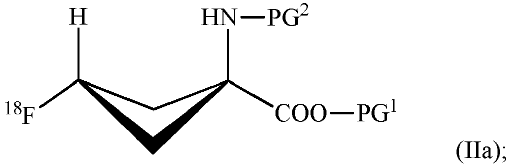 and, said compound of Formula III is a compound of Formula IIIa: 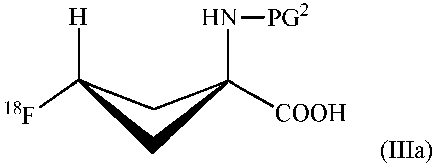 wherein PG¹ and PG² are as described hereinabove.

In some embodiments the method of present invention additionally includes a step following the reacting step and before the formulating step of purifying the reaction mixture obtained in the reacting step to obtain substantially pure [¹⁸F]FACBC.

The term "substantially" as used in "substantially pure" takes the meaning as presented above. The term "substantially pure" as used in the context of [¹⁸F]FACBC encompasses completely pure [¹⁸F]FACBC or [¹⁸F]FACBC that is sufficiently pure to be suitable for use as a PET tracer. The term "suitable for use as a PET tracer" means that the [¹⁸F]FACBC product is suitable for intravenous administration to a mammalian subject followed by PET imaging to obtain one or more clinically-useful images of the location and/or distribution of [¹⁸F]-FACBC.

A suitable purifying step comprises:
(i) carrying out a first purification step comprising passing said reaction mixture through a hydrophilic lipophilic balanced (HLB) solid phase; and,
(ii) optionally carrying out a second purification step comprising passing said reaction mixture through an alumina solid phase.

In certain embodiments of the present invention said purifying step can be said to consist essentially of the above-defined steps. In particular, the purifying step does not require that the reaction mixture is passed through an ion retardation column. This is a notable distinction over the prior art methods where this is a required step in order to remove ions and to neutralise the reaction mixture (e.g. as described by McConathy et al (2003 Appl Radiat Isotop; 58: 657-666), and in EP20172580029 (A)). As such, the method of the present invention is simplified over the prior art methods and as such is more suitable for automation.

In a preferred embodiment the method of the invention is carried out on an automated synthesis apparatus. By the term "automated synthesis apparatus" is meant an automated module based on the principle of unit operations as described by Satyamurthy et al (1999 Clin Positr Imag; 2(5): 233-253). The term 'unit operations" means that complex processes are reduced to a series of simple operations or reactions, which can be applied to a range of materials. Such automated synthesis apparatuses are preferred for the method of the present invention especially when a radiopharmaceutical composition is desired. They are commercially available from a range of suppliers (Satyamurthy *et al*, above), including: GE Healthcare; CTI Inc; Ion Beam Applications S.A. (Chemin du Cyclotron 3, B-1348 Louvain-La-Neuve, Belgium); Raytest (Germany) and Bioscan (USA).

A commercial automated synthesis apparatus also provides suitable containers for the liquid radioactive waste generated as a result of the radiopharmaceutical preparation. Automated synthesis apparatuses are not typically provided with radiation shielding, since they are designed to be employed in a suitably configured radioactive work cell. The radioactive work cell provides suitable radiation shielding to protect the operator from potential radiation dose, as well as ventilation to remove chemical and/or radioactive vapours. The automated synthesis apparatus preferably comprises a cassette. By the term "cassette" is meant a piece of apparatus designed to fit removably and interchangeably onto an automated synthesis apparatus, in such a way that mechanical movement of moving parts of the synthesizer controls the operation of the cassette from outside the cassette, i.e. externally. Suitable cassettes comprise a linear array of valves, each linked to a port where reagents or vials can be attached, by either needle puncture of an inverted septum-sealed vial, or by gas-tight, marrying joints. Each valve has a male-female joint which interfaces with a corresponding moving arm of the automated synthesis apparatus. External rotation of the arm thus controls the opening or closing of the valve when the cassette is attached to the automated synthesis apparatus. Additional moving parts of the automated synthesis apparatus are designed to clip onto syringe plunger tips, and thus raise or depress syringe barrels.

The cassette is versatile, typically having several positions where reagents can be attached, and several suitable for attachment of syringe vials of reagents or chromatography cartridges (e.g. for SPE). The cassette always comprises a reaction vessel. Such reaction vessels are preferably 0.5 to 10 mL, more preferably 0.5 to 5 mL and most preferably 0.5 to 4 mL in volume and are configured such that 3 or more ports of the cassette are connected thereto, to permit transfer of reagents or solvents from various ports on the cassette. Preferably the cassette has 15 to 40 valves in a linear array, most preferably 20 to 30, with 25 being especially preferred. The valves of the cassette are preferably each identical, and most preferably are 3-way valves. The cassettes are designed to be suitable for radiopharmaceutical manufacture and are therefore manufactured from materials which are of pharmaceutical grade and ideally also are resistant to radiolysis.

Preferred automated synthesis apparatuses for use with the present invention comprise a disposable or single use cassette which comprises all the reagents, reaction vessels and apparatus necessary to carry out the preparation of a given batch of radiofluorinated radiopharmaceutical. The cassette means that the automated synthesis apparatus has the flexibility to be capable of making a variety of different radiopharmaceuticals with minimal risk of cross-contamination, by simply changing the cassette. The cassette approach also has the advantages of: simplified set-up hence reduced risk of operator error; improved GMP (Good Manufacturing Practice) compliance; multi-tracer capability; rapid change between production runs; pre-run automated diagnostic checking of the cassette and reagents; automated barcode cross-check of chemical reagents *vs* the synthesis to be carried out; reagent traceability; single-use and hence no risk of cross-contamination, tamper and abuse resistance.

The following example serves to further illustrate the invention.

### Brief Description of the Examples

Example 1 describes a method to obtain the composition of the present invention.

### List of Abbreviations used in the Examples

- ATR: attenuated total reflectance
- DTGS: deuterated triglycine sulphate
- [¹⁸F]FACBC: 1-amino-3-[¹⁸F]fluorocyclobutane-1-carboxylic acid
- FT-IR: Fourier transform infrared
- K222: Kryptofix 222
- MeCN: acetonitrile
- MeOH: methanol
- QMA: quaternary methyl ammonium
- RCY: radiochemical yield
- SPE: solid-phase extraction
- TLC: thin layer chromatography
- UV: ultraviolet

### Examples

All reagents and solvents were purchased from Merck and used without further purification. The [¹⁸F]FACBC precursor; *Syn*-1-(N-(tert-butoxycarbonyl)amino)-3-[[(trifluoromethyl)sulfonyl]oxy]-cyclobutane-1-carboxylic acid ethyl ester was obtained from GE Healthcare. The Oasis HLB plus cartridge and the Sep-Pak cartridges: QMA light Plus (K₂CO₃ form), tC18 light, Alumina N light were purchased from Waters (Milford, MA, USA). A Capintec NaI ion chamber was used for all radioactive measurements (model CRC15R). Radio-thin layer chromatography (radio-TLC) was performed on a Packard instant imager using pre-coated plates of silica gel (Merck 60F₂₅₄).

### Example 1: Synthesis and Formulation of [¹⁸F]FACBC Composition of the Invention

No-carrier-added [¹⁸F]fluoride was produced via the ¹⁸O(p,n)¹⁸F nuclear reaction on a GE PETtrace 6 cyclotron (Norwegian Cyclotron Centre, Oslo). Irradiations were performed using a dual-beam, 30µA current on two equal Ag targets with HAVAR foils using 16.5 MeV protons. Each target contained 1.6 ml of ≥ 96% [¹⁸O]water (Marshall Isotopes). Subsequent to irradiation and delivery to a hotcell, each target was washed with 1.6 ml of [¹⁶O]water (Merck, water for GR analysis), giving approximately 2-5 Gbq in 3.2 ml of [¹⁶O]water.

All radiochemistry was performed on a commercially available GE FASTlab^{™} with a single-use cassette. Each cassette is built around a one-piece-moulded manifold with 25 three-way stopcocks, all made of polypropylene. Briefly, the cassette includes a 5 ml reactor (cyclic olefin copolymer), one 1 ml syringe and two 5 ml syringes, spikes for connection with five prefilled vials, one water bag (100 ml) as well as various SPE cartridges and filters. Fluid paths are controlled with nitrogen purging, vacuum and the three syringes. The fully automated system is designed for single-step fluorinations with cyclotron-produced [¹⁸F]fluoride. The FASTlab was programmed by the software package in a step-by-step time-dependent sequence of events such as moving the syringes, nitrogen purging, vacuum, and temperature regulation. Synthesis of [¹⁸F]FACBC followed the three general steps: (a) [¹⁸F]fluorination, (b) hydrolysis of protection groups and (c) SPE purification.

Vial A contained K₂₂₂ (156 µmol), K₂CO₃ (60.8 µmol) in 79.5% (v/v) MeCN_{(aq)} (1105 µl). Vial B contained 4M HCl. Vial C contained MeCN. Vial D contained precursor (123.5 µmol) in its dry form (stored below -5 °C until cassette assembly). Vial E contained 2 M NaOH (4.1 ml). The 30 ml product collection glass vial was filled with 200 mM citrate buffer (10 ml). Aqueous [¹⁸F]fluoride (1-1.5 ml, 100-200 Mbq) was passed through the QMA and into the ¹⁸O-H₂O recovery vial. The QMA was then flushed with MeCN and sent to waste. The trapped [¹⁸F]fluoride was eluted into the reactor using eluent from vial A (730 µl) and then concentrated to dryness by azeotropic distillation with acetonitrile (80 µl, vial C). Approximately 1.7 ml of MeCN was mixed with precursor in vial D from which 1.0 ml of the dissolved precursor (corresponds to 72.7 mmol precursor) was added to the reactor and heated for 3 min at 85°C. The reaction mixture was diluted with water and sent through the tC 18 cartridge. Reactor was washed with water and sent through the tC18 cartridge. The labelled intermediate, fixed on the tC 18 cartridge was washed with water, and then incubated with 2M NaOH (2.0 ml) for 5 min. The labelled intermediate (without the ester group) was eluted off the tC 18 cartridge into the reactor using water. The BOC group was hydrolysed by adding 4M HCl (1.4 ml) and heating the reactor for 5 min at 60 °C. The reactor content with the crude [¹⁸F]FACBC was sent through the HLB and Alumina cartridges and into the 30 ml product vial. The HLB and Alumina cartridges were washed with water (9.1 ml total) and collected in the product vial. Finally, 2M NaOH (0.9 ml) and water (2.1 ml) was added to the product vial, giving the purified formulation of [¹⁸F]FACBC with a total volume of 26 ml. Radiochemical purity was measured by radio-TLC using a mixture of MeCN:MeOH:H₂O:CH₃COOH (20:5:5:1) as the mobile phase. The radiochemical yield (RCY) was expressed as the amount of radioactivity in the [¹⁸F]FACBC fraction divided by the total used [¹⁸F]fluoride activity (decay corrected). Total synthesis time was 43 min.

## Claims

1. An automated method to obtain a radiopharmaceutial composition of ¹⁸F-1-amino-3-fluoro-cyclobutane-1-carboxylic acid (¹⁸F-FACBC) wherein said composition;
(a) comprises 50-100 mM citrate buffer; and,
(b) has a pH of 4.0 - 5.0;
wherein said method comprises:
(i) reacting with a suitable source of [¹⁸F]fluoride a precursor compound of Formula I: 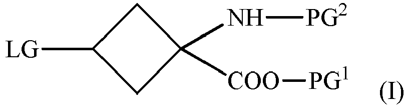 wherein:
LG is a leaving group;
PG¹ is a carboxy protecting group; and,
PG² is an amine protecting group;
to obtain a compound of Formula II: 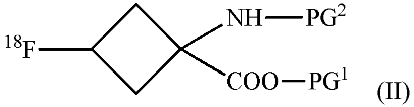 wherein PG¹ and PG² are as defined for Formula I;
(ii) reacting said compound of Formula II with a PG¹ deprotecting agent to obtain a compound of Formula III: 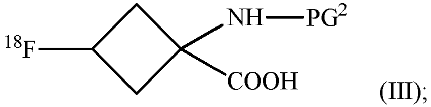 wherein PG² is as defined for Formula I;
(iii) reacting said compound of Formula III with a PG² deprotecting agent to obtain a crude product containing ¹⁸F-FACBC;
(iv) carrying out a purification step comprising passing said crude product through a hydrophilic lipophilic balanced (HLB) solid phase to obtain [¹⁸F]-FACBC, wherein said crude product is not passed through an ion retardation column; and
(v) formulating said [¹⁸F]-FACBC with citrate buffer to obtain said pharmaceutical composition.

2. The method as defined in Claim 1 wherein said [¹⁸F]-FACBC is trans-1-amino-3-[¹⁸F]-fluorocyclobutanecarboxylic acid (*anti*-[¹⁸F]-FACBC): 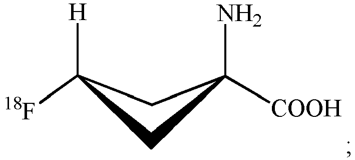 said compound of Formula I is a compound of Formula Ia: 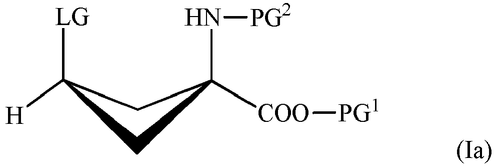 said compound of Formula II is a compound of Formula IIa: 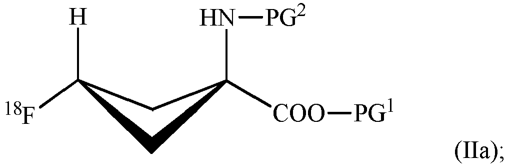 and, said compound of Formula III is a compound of Formula IIIa: 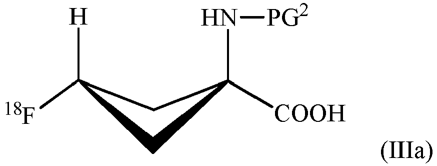 wherein PG¹ and PG² are as defined in Claim 1 for Formula I.

3. The method as defined in any one of Claims 1-2 wherein the pharmaceutical composition comprises 60-90 mM citrate buffer.

4. The method as defined in any one of Claims 1-3 wherein the pharmaceutical composition has a pH of 4.1-4.5.

5. The method as defined in any one of Claims 1-4 wherein the pharmaceutical composition has an end of synthesis (EOS) radioactive concentration (RAC) of at least 1000 MBq/mL.

6. The method as defined in any one of Claims 1-5 wherein the pharmaceutical composition comprises not more than 150 µg/mL 1-amino-3-hydroxyl-cyclobutane-1-carboxylic acid (hydroxyl-ACBC).

7. The method as defined in any one of Claims 1-6 wherein the pharmaceutical composition comprises not more than 0.15 µg/mL 1-amino-3-fluoro-cyclobutane-1-carboxylic acid (FACBC).

8. The method as defined in any one of Claims 1-7 wherein the pharmaceutical composition comprises not more than 2.0 µg/mL 1-amino-3-chloro-cyclobutane-1-carboxylic acid (chloro-ACBC).

9. The method as defined in any one of Claims 1-8 wherein the pharmaceutical composition does not comprise a radiostabiliser.

10. The method as defined in any one of claims 1-9 which is carried out on an automated synthesis apparatus.

11. The method as defined in any one of Claims 1-10 wherein said PG¹ deprotecting agent is NaOH.

12. The method as defined in any one of Claims 1-11 wherein said PG² deprotecting agent is HCl.

## Patentansprüche

1. Automatisiertes Verfahren zum Erhalt einer radiopharmazeutischen Zusammensetzung von ¹⁸F-1-Amino-3-fluorcyclobutan-1-carbonsäure (¹⁸F-FACBC), wobei die Zusammensetzung
(a) 50-100 mM Citratpuffer umfasst und
(b) einen pH-Wert von 4,0-5,0 aufweist;
wobei das Verfahren Folgendes umfasst:
(i) Umsetzen einer Vorläuferverbindung der Formel I: 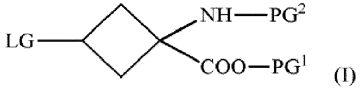 wobei:
LG für eine Abgangsgruppe steht;
PG¹ für eine Carboxyschutzgruppe steht und
PG² für eine Aminoschutzgruppe steht;
mit einer geeigneten Quelle von [¹⁸F]-Fluorid zum Erhalt einer Verbindung der Formel II: 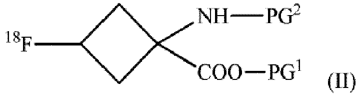 wobei PG¹ und PG² wie für Formel I definiert sind;
(ii) Umsetzen der Verbindung der Formel II mit einem PG¹-Entschützungsmittel zum Erhalt einer Verbindung der Formel III: 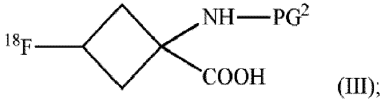 wobei PG² wie für Formel I definiert ist;
(iii) Umsetzen der Verbindung der Formel III mit einem PG²-Entschützungsmittel zum Erhalt eines Rohprodukts, das ¹⁸F-FACBC enthält;
(iv) Durchführen eines Reinigungsschritts, bei dem man das Rohprodukt durch eine HLB-Festphase (HLB = hydrophilic lipophilic balanced) führt, um [¹⁸F]-FACBC zu erhalten, wobei das Rohprodukt nicht durch eine Ionenverzögerungssäule geführt wird; und
(v) Formulieren der [¹⁸F]-FACBC mit Citratpuffer zum Erhalt der pharmazeutischen Zusammensetzung.

2. Verfahren gemäß Anspruch 1, wobei es sich bei der [¹⁸F]-FACBC um trans-1-Amino-3-[¹⁸F]-fluorcyclo-butan-1-carbonsäure (anti-[¹⁸F]-FACBC) handelt: 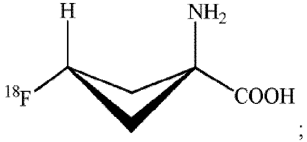 es sich bei der Verbindung der Formel I um eine Verbindung der Formel Ia handelt: 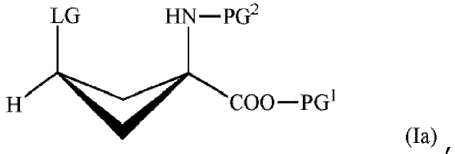 es sich bei der Verbindung der Formel II um eine Verbindung der Formel IIa handelt: 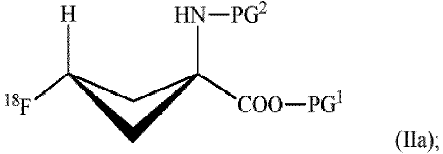 und es sich bei der Verbindung der Formel III um eine Verbindung der Formel IIIa handelt: 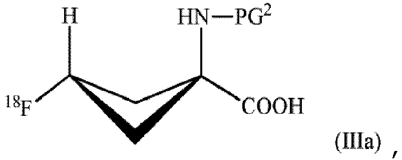 wobei PG¹ und PG² wie in Anspruch 1 für Formel I definiert sind.

3. Verfahren gemäß einem der Ansprüche 1-2, wobei die pharmazeutische Zusammensetzung 60-90 mM Citratpuffer umfasst.

4. Verfahren gemäß einem der Ansprüche 1-3, wobei die pharmazeutische Zusammensetzung einen pH-Wert von 4,1-4,5 aufweist.

5. Verfahren gemäß einem der Ansprüche 1-4, wobei die pharmazeutische Zusammensetzung eine radioaktive Konzentration (RAC) am Ende der Synthese (EOS) von mindestens 1000 MBq/ml aufweist.

6. Verfahren gemäß einem der Ansprüche 1-5, wobei die pharmazeutische Zusammensetzung nicht mehr als 150 µg/ml 1-Amino-3-hydroxylcyclobutan-1-carbonsäure (Hydroxyl-ACBC) umfasst.

7. Verfahren gemäß einem der Ansprüche 1-6, wobei die pharmazeutische Zusammensetzung nicht mehr als 0,15 µg/ml 1-Amino-3-fluorcyclobutan-1-carbonsäure (FACBC) umfasst.

8. Verfahren gemäß einem der Ansprüche 1-7, wobei die pharmazeutische Zusammensetzung nicht mehr als 2,0 mg/ml 1-Amino-3-chlorcyclobutan-1-carbonsäure (Chlor-ACBC) umfasst.

9. Verfahren gemäß einem der Ansprüche 1-8, wobei die pharmazeutische Zusammensetzung keinen Radiostabilisator umfasst.

10. Verfahren gemäß einem der Ansprüche 1-9, das auf einer automatisierten Syntheseapparatur durchgeführt wird.

11. Verfahren gemäß einem der Ansprüche 1-10, wobei es sich bei dem PG¹-Entschützungsmittel um NaOH handelt.

12. Verfahren gemäß einem der Ansprüche 1-11, wobei es sich bei dem PG²-Entschützungsmittel um HCl handelt.

## Revendications

1. Procédé automatisé pour obtenir une composition radiopharmaceutique d'acide ¹⁸F-1-amino-3-fluoro-cyclobutane-carboxylique (¹⁸F-FACBC), ladite composition ;
(a) comprenant 50 à 100 mM de tampon citrate ; et,
(b) possédant un pH de 4,0 à 5,0 ;
ledit procédé comprenant :
(i) la mise en réaction, avec une source appropriée de [¹⁸F]fluorure, d'un composé précurseur de Formule I : 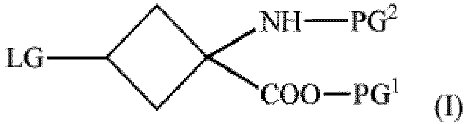
LG étant un groupe partant ;
PG¹ étant un protecteur de carboxy ; et,
PG² étant un groupe protecteur d'amine ;
pour obtenir un composé de Formule II : 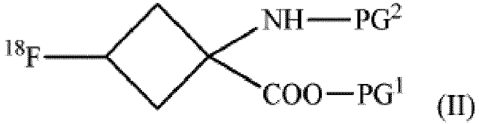 PG¹ et PG² étant tels que définis pour la Formule I ;
(ii) la mise en réaction dudit composé de Formule II avec un agent de déprotection de PG¹ pour obtenir un composé de Formule III : 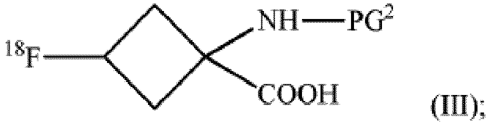 PG² étant tel que défini pour la formule I ;
(iii) la mise en réaction dudit composé de Formule III avec un agent de déprotection de PG² pour obtenir un produit brut contenant ¹⁸F-FACBC ;
(iv) la mise en œuvre d'une étape de purification comprenant le passage dudit produit brut à travers une phase solide équilibrée hydrophile lipophile (HLB) pour obtenir [¹⁸F]-FACBC, ledit produit brut n'étant pas passé à travers une colonne de retardement d'ions ; et
(v) la formulation dudit [¹⁸F]-FACBC avec un tampon citrate pour obtenir ladite composition pharmaceutique.

2. Procédé tel que défini dans la revendication 1, ledit [¹⁸F]-FACBC étant l'acide trans-1-amino-3-[¹⁸F]-fluorocyclobutanecarboxylique (*ant*-[¹⁸F]-FACBC) : 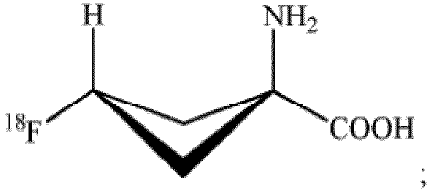 ledit composé de Formule I étant un composé de Formule la : 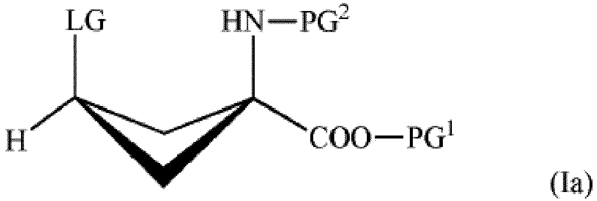 ledit composé de Formule II étant un composé de Formule IIa : 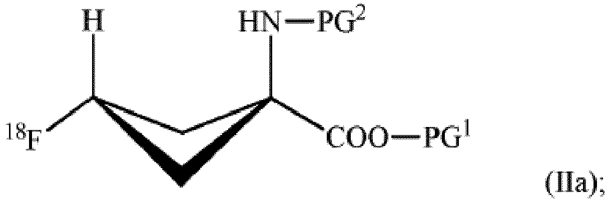 et, ledit composé de Formule III étant un composé de Formule IIIa : 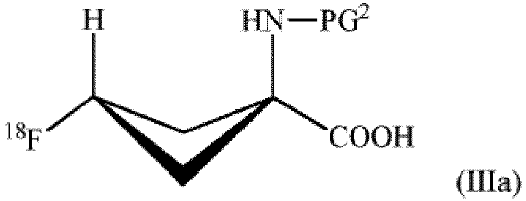 PG¹ et PG² étant tels que définis dans la revendication 1 pour la Formule I.

3. Procédé tel que défini dans l'une quelconque des revendications 1 et 2, la composition pharmaceutique comprenant 60 à 90 mM de tampon citrate.

4. Procédé tel que défini dans l'une quelconque des revendications 1 à 3, la composition pharmaceutique possédant un pH de 4,1 à 4,5.

5. Procédé tel que défini dans l'une quelconque des revendications 1 à 4, la composition pharmaceutique possédant une concentration radioactive (RAC) en fin de synthèse (EOS) d'au moins 1 000 MBq/mL.

6. Procédé tel que défini dans l'une quelconque des revendications 1 à 5, la composition pharmaceutique ne comprenant pas plus de 150 µg/mL d'acide 1-amino-3-hydroxyl-cyclobutane-1-carboxylique (hydroxyl-ACBC).

7. Procédé tel que défini dans l'une quelconque des revendications 1 à 6, la composition pharmaceutique ne comprenant pas plus de 0,15 µg/mL d'acide 1-amino-3-fluoro-cyclobutane-1-carboxylique (FACBC).

8. Procédé tel que défini dans l'une quelconque des revendications 1 à 7, la composition pharmaceutique ne comprenant pas plus de 2,0 µg/mL d'acide 1-amino-3-chloro-cyclobutane-1-carboxylique (chloro-ACBC).

9. Procédé tel que défini dans l'une quelconque des revendications 1 à 8, la composition pharmaceutique ne comprenant pas de radiostabilisant.

10. Procédé tel que défini dans l'une quelconque des revendications 1 à 9 qui est mis en œuvre sur un appareil de synthèse automatisée.

11. Procédé tel que défini dans l'une quelconque des revendications 1 à 10, ledit agent de déprotection de PG¹ étant NaOH.

12. Procédé tel que défini dans l'une quelconque des revendications 1 à 11, ledit agent de déprotection de PG² étant HCl.
